# EUROPEAN PATENT SPECIFICATION

(11) **EP 4 398 964 B1**
(45) Date of publication and mention of the grant of the patent: **15.07.2026**
(21) Application number: 22868191.2
(22) Date of filing: 12.09.2022
(51) Int. Cl.: A61M 11/00, A61M 11/02, A61M 15/00, A61M 15/06, A61K 9/00

(54) **INHALER**
INHALATOR
INHALATEUR

(30) Priority: 12.09.2021 US 202163243198 P; 12.09.2021 US 202163243200 P
(43) Date of publication of application: 17.07.2024
(73) Proprietor: Niconox LLC, Monmouth Junction, NJ 08852 (US)
(72) Inventor: VELAGA, Sitaram, Monmouth Junction, NJ 08852 (US); STADERMAN, David, Monmouth Junction, NJ 08852 (US); GONDA, Igor, Dennis, MA 02638 (US)
(74) Representative: Herrero & Asociados, S.L.
(86) International application number: PCT/US2022/043262
(87) International publication number: WO 2023/039266

(56) References cited:
- WO-A1-00/50112
- WO-A1-2004/078244
- WO-A1-2009/013501
- WO-A1-2009/135729
- WO-A1-2015/013329
- WO-A1-2019/071008
- WO-A2-2013/116568
- US-A- 4 846 168
- US-A- 4 945 929
- US-A1- 2005 183 718
- US-A1- 2014 190 496
- US-A1- 2014 190 496
- US-A1- 2014 261 474
- US-A1- 2020 139 057
- US-A1- 2021 228 822
- US-B1- 9 016 274
- US-B2- 10 456 535
- US-B2- 9 084 660
- US-B2- 9 084 660
- US-B2- 9 302 060

## Description

### FIELD OF INVENTION

The subject disclosure relates to devices and formulations to deliver aerosols of active pharmaceutical ingredient (API), e.g., nicotine and nicotine salts, for inhalation.

### BACKGROUND

Delivery of nicotine by inhalation of tobacco smoke has been practiced by humans since c.5000 B.C., and widespread since at least the 16^{th} century, if not much earlier. The harmful effects of tobacco smoking are not primarily due to nicotine itself, but rather resulting from the many of the other ingredients that result from the combustion of tobacco smoke. Quitting smoking is difficult due to the highly addictive nature of nicotine, as nicotine elicits several effects that the users may find attractive, including euphoria, suppression of depression, appetite reduction, improved concentration and improved memory recall.

Nicotine is approved as a pharmaceutical treatment - nicotine replacement therapy (NRT) - to aid in quitting tobacco smoking and/or reducing the craving for cigarettes or nicotine. The routes of the approved product administration include oral, nasal, transdermal, buccal and inhaled. These products show a variety of systemic blood pharmacokinetics of nicotine but none of them achieve speeds of creating an early high concentration of nicotine similar to smoking cigarettes. NRT can also be successful in preventing relapses in smokers who quit.

It is known that the key reason for smokers' inability to quit is succumbing to their nicotine cravings, which have been satisfied from the physiological response to smoking a cigarette. Unless this craving is rapidly satisfied, NRT can fail. Slow delivery of nicotine, even if ultimately the resulting blood levels of nicotine can be very high, does not appear to be able to prevent the craving for cigarettes effectively. The acute tolerance to nicotine's ability to elicit the central nervous system (CNS) effects has been reported to be very rapidly developed (Benowitz, 1996; Porchet et al., 1987). This may explain the importance of the early pharmacokinetic (PK) profiles of NRTs, i.e., the apparent need for an early peak in nicotine to reach the brain for the attendant CNS effects.

While some of the electronic cigarettes based on the principle of vaporization of mixture of nicotine with organic solvents approach the rapid pharmacokinetics of nicotine from cigarettes, there are major concerns about the safety of these products due to the reported battery explosions as well as the toxicity of the emissions containing a mixture of organic solvents, their contaminants, and the degradations products from the storage and aerosolization. Also, some users of electronic cigarettes do not perceive the same physiological response to vaping as they do when smoking traditional cigarettes.

There thus remains a need for effective and safe NRT.

Additionally, nicotine, as an API, is known to treat a variety of conditions. There remains a need for alternative ways of delivering nicotine for nicotine therapy.

Further, nicotine is just an example of an API that could be administered via the pulmonary route, via an inhaler. There remains a need for safe and alternative ways of administering such APIs.

Soft mist inhalers are known from US 9 084 660 B2, US 2005/183718 A1, WO 2019/071008 A1 and US 2021/228822 A1.

### SUMMARY

According to the invention, there is provided an inhaler according to claim 1. The dependent claims define embodiments of the invention as claimed.

One aspect of the disclosed subject matter provides an inhaler for delivering an API in aerosol form. The inhaler includes a container that includes an API and one or more pharmaceutically acceptable carriers; a nozzle, valve, or outlet in communication with the container to deliver an aerosol; an insert located downstream of the nozzle, valve or outlet.

In an exemplary embodiment, the insert is located from about 1 to about 10 mm downstream from the nozzle, valve, or outlet. In one embodiment, the insert includes a centrally located orifice. In one embodiment, the insert provides obstructions along from about 10% to about 90%, or from about 25% to about 75% of a total cross-sectional area along an airpath downstream of the nozzle, valve, or outlet.

In an exemplary embodiment, the container includes API in dry powder form. In an exemplary embodiment, the container includes API is in an aqueous solution. In an exemplary embodiment, the aqueous solution includes an alcohol (e.g., ethanol from about 25 to about 75 v/v%).

In an exemplary embodiment, the inhaler emits an aerosol dose including the API in which at least 30%, of the emitted dose has aerodynamic diameters between 0.5 - 3.5 micrometers. In another exemplary embodiment, the aerosol dose is emitted at inspiratory flow rates between 5 - 140 L/min. In another exemplary embodiment, at least 40% the emitted dose is contained in droplets with aerodynamic sizes less than about 4 microns.

In one exemplary embodiment, the API is nicotine or a salt thereof. In one exemplary embodiment, the API is in the form of a nicotine salt, such as a citrate salt or a sulphate salt. In one exemplary embodiment, from about 0.01 mg to about 1 mg of nicotine (free base basis) is delivered per actuation.

Another aspect of the subject disclosure provides a method of targeting delivery of an API to the lungs of subject. The method includes administering to the subject an inhaler that includes a container that includes an API and one or more pharmaceutically acceptable carriers; a nozzle, valve, or outlet in communication with the container to deliver an aerosol; an insert located downstream of the nozzle, valve or outlet, the insert disposed in a mouthpiece of the inhaler. In one exemplary embodiment, the API is nicotine or a salt thereof.

Another aspect of the subject disclosure provides a method for performing Nicotine Replacement Therapy (NRT) on a subject to reduce or prevent consumption of cigarettes and other forms of tobacco. The method includes administering to the subject an inhaler that includes a container that includes nicotine or salt thereof and one or more pharmaceutically acceptable carriers; a nozzle, valve, or outlet in communication with the container to deliver an aerosol; and an insert located downstream of the nozzle, valve or outlet in a mouthpiece of the inhaler.

Another aspect of the subject disclosure provides an inhaler capable of generating an aerosol that includes one or more inserts located downstream from a location where the aerosol is emitted. In one embodiment, the one or more inserts are located in a mouthpiece of the inhaler. In one embodiment, the inhaler is a soft mist inhaler (SMI). In one embodiment, the SMI forms intersecting jets to form aerosol droplets. In one embodiment, the inhaler is a propellant-driven inhaler. In one embodiment, the inhaler is a dry powder inhaler (DPI). In one embodiment, the inhaler includes a nebulizer. In one embodiment, the nebulizer is selected from a jet nebulizer, an ultrasonic nebulizer, a mesh nebulizer.

In one embodiment, the insert is located from about 1 to about 10 mm downstream from the location where the aerosol is generated. In one embodiment, the insert includes a centrally located orifice. In one embodiment, the insert provides obstructions along from about 10% to about 90% of the total mouthpiece cross-sectional area along an airpath downstream of the location where the aerosol is emitted.

Another aspect of the subject disclosure provides an inhaler that includes a container that includes nicotine or a salt thereof and one or more pharmaceutically acceptable carriers; a nozzle, valve, or outlet in communication with the container to deliver an aerosol suitable for pulmonary administration to a human; and a mouthpiece sized for administration to a mouth of the human. In one embodiment, the inhaler is a soft mist inhaler (SMI). In one embodiment, the SMI forms intersecting jets to form aerosol droplets. In one embodiment, the inhaler is a propellant-driven inhaler. In one embodiment, the inhaler is a dry powder inhaler (DPI). In one embodiment, the inhaler includes a nebulizer. In one embodiment, the nebulizer is selected from a jet nebulizer, an ultrasonic nebulizer, a mesh nebulizer.

### BRIEF DESCRIPTION OF THE DRAWINGS

FIG. 1 depicts an inhaler according to an exemplary embodiment of the subject disclosure, which is also an embodiment of the invention as claimed.
FIGS. 2A-2H depicts exemplary inserts of the subject disclosure.
FIG. 3 depicts the percentage of total dosage recovered at various stages of delivery for a 0% ethanol formulation as described in Example 3, using inserts of the subject disclosure.
FIG. 4 depicts the percentage of total dosage recovered at various stages of delivery for a 25% ethanol formulation as described in Example 3, using inserts of the subject disclosure.
FIG. 5 depicts the percentage of total dosage recovered at various stages of delivery for a 75% ethanol formulation as described in Example 3, using inserts of the subject disclosure.
FIG. 6 summarizes the total dose recovered for various ethanol formulations, grouped by delivery areas using no insert.
FIG. 7 summarizes the total dose recovered for various ethanol formulations, grouped by delivery areas using insert 4.
FIG. 8 summarizes the total dose recovered for various ethanol formulations, grouped by delivery areas using insert 5.
FIG. 9 summarizes the total dose recovered for various ethanol formulations, grouped by delivery areas using insert 7.
FIG. 10 depicts the mass of API deposited based on placement of insert 4 from the nozzle as described in Example 3.
FIG. 11 depicts the mass of API deposited based on placement of insert 5 from the nozzle as described in Example 3.
FIG. 12 depicts the mass of API deposited based on placement of insert 7 from the nozzle as described in Example 3.
FIG. 13 depicts the lung/throat deposition ratio for various inserts (and no insert) at various ethanol concentrations as described in Example 3.
FIG. 14 depicts the amount of API recovered at various stages employing formulations containing various strengths of API.
FIG. 15 depicts the percentage of API recovered at various stages at various stages employing formulations containing various strengths of API.
FIG. 16 depicts the fine particle distribution as a function of nicotine concentration, as described in Example 6.

### DETAILED DESCRIPTION

For purposes of brevity, the presently disclosed subject matter will be described in connection with nicotine as an exemplary API. It is understood, however, that the disclosed subject matter can be applied to any API.

In addition to the APIs in the inhalation product, the presently disclosed formulations can, in certain embodiments, contain one or more other ingredients that are beneficial for the performance of the product. These substances generally are not actively participating in the biological activity but may be necessary as the carriers for the therapeutic substances. For example, in the case of nebulized therapeutics and vaccines, the carrier may be water and various substances to adjust or stabilize the properties such as acidity, osmolarity, taste or stability of the active ingredient. Other solvents such as ethanol and other alcohols may be also used, as well as surface active agents. In some situations, it may be necessary to use bacterial preservatives.

Encapsulation of the therapeutic substances can also be employed to improve drug targeting and modulating its pharmacokinetics, for example encapsulation in liposomes and other phospholipid containing vesicles, nanoparticles and other such formulation ingredients known in the art. Suspensions of solid particles in liquids or emulsions can be also used which may need stabilization with surface active agents, or the densities are adjusted such that sedimentation or creaming does not cause instability of the product.

Formulations of the subject disclosure can include nicotine (free base) and/or nicotine salts as the active agent. Nicotine salts are formed by protonating the free base nicotine with an acid, such as sulphuric, glycolic, pyruvic, lactic, levulinic, fumaric, succinic, benzoic, salicylic, malic, tartaric, and citric acids. See Nicotine Tob Res. 2020 Jul; 22(7):1239-1243. This reduces the pH of the formulation (e.g., pH < 6.0), as compared to free-base nicotine. This allows higher concentrations of nicotine to be administered, if desired, while not inducing an irritating "throat hit." Nicotine salts are described in U.S. Patent No. 9,215,895.

Nicotine can also be also administered with the use of pressurized propellants that include but are not limited to chlorofluorocarbons and hydrofluoroalkanes and their mixtures. Such formulations may also include other liquids, such as ethanol. Alternatively, in other embodiments, the formulations do not include pressurized propellants and consist, or consist essentially of, nicotine and an appropriate solvent, such as water or aqueous ethanol solutions.

The inhalation products may also be in the form of dry powders administered by dry powder inhalers. These can contain particles containing the active ingredient(s), i.e. nicotine and/or nicotine salts, but they may also have other substances (e.g., solvents, carriers and other types of excipients) incorporated to improve the product attributes to the desired performance such as particle size distribution, morphology, density, shape, dispersibility and stability for the optimum performance of the product. For example, carrier particles can be employed to assist in the delivery of small respirable active ingredients in precise quantities to the desired parts of the respiratory tract. The ingredients of such dry powders include, but are not limited to, sugars such as lactose, mannitol, sucrose or trehalose, amino acids such as leucine and isoleucine, phospholipids, cholesterol, magnesium stearate and so on.

In addition to NRT, the instantly disclosed formulations can alternatively be administered to treat systemic diseases via absorption of nicotine from the respiratory tract into the systemic circulation. Generally, the large surface area of the bronchoalveolar regions with its permeable properties is the desired deposition region for this purpose. Examples of the treatments and prophylaxis of systemic diseases include but are not limited to Parkinson's Disease, Alzheimer's Disease, depression, anxiety, schizophrenia, attention deficit hyperactivity disorder, pain, and obesity. Other exemplary embodiments provide a method of activating nicotinic acetylcholine receptors (nAChRs), and the administered formulations are used to treat conditions that are improved or ameliorated upon activation of NAChRs receptors. See e.g., Nicotine as Therapy, PLoS Biol. 2004 Nov; 2(11):e404.

The delivery of nicotine by inhalation in a form leading to an early high nicotine peak would be an attractive method to help tobacco smokers to quit as well as to deliver nicotine for other therapeutic purposes such as to treat depression, memory loss, lack of concentration and excessive appetite. There are also encouraging reports about the potential benefits of nicotine to treat neurogenerative diseases including Alzheimer's and Parkinson's disease. Such delivery needs to be well tolerated and safe. The product should have a minimum content of any potentially harmful ingredients that may components of the product, or enter the product either during manufacturing or as a result of storage and use of the product.

Nicotine can be a highly irritating substance. Although smokers are known to develop tolerance to the side effects of nicotine including self-limiting coughing reflex, it is nevertheless preferential to design a nicotine inhaler that would emulate as much as possible the delivery of nicotine in a way that is similar to the regional distribution of deposited nicotine following inhalation of cigarette smoke. This may also provide smokers with a similar sensation to smoking cigarettes to assist in tobacco smoking cessation.

Satisfying the craving for nicotine is a complex phenomenon that depends on many factors including the biochemical and physiological effects of nicotine on the brain but also other sensory perceptions including those resulting from nicotine entering into the respiratory tract. Nevertheless, generally rapid attainment of high nicotine peaks in the blood stream is associated with better and faster elimination or reduction of craving for cigarettes. To achieve this type of kinetic profile of nicotine in the user's blood stream, it is desirable to present nicotine in the form of particles or droplets that deposit primarily in the small airways and alveoli with their highly permeable membranes and very large surface area. The key attributes that control this type of preferential deposition in the distal parts of the respiratory tract while minimizing the deposition in the oropharyngeal cavity and the large airways are the droplet or particle size, shape and velocity as well as the placement of the aerosol containing nicotine in the volume of inhaled air; e.g., releasing a small volume of nicotine in small droplets early at the beginning of a slow inspiration can result in deposition in small airways and alveoli. Conversely, inhaling rapidly large droplets of nicotine aerosol released from an inhaler late in the inspiration can enhance deposition in the oropharynx and large airways.

Exemplary embodiments of the subject disclosure achieve such deposition patterns by administration of droplets of nicotine solutions in which at least 10% or 20% of the emitted dose has aerodynamic diameters between 0.05 - 40 micrometers that are inhaled at inspiratory flow rates between 1-300 L/min. Other exemplary embodiments of the subject disclosure achieve such preferential deposition patterns by administration of droplets of nicotine solutions in which at least 30%, 40%, or 50% of the emitted dose has aerodynamic diameters between 0.5 - 3.99 micrometers that are inhaled at inspiratory flow rates between 10-60 or 5-140 L/min.

This will enable lower mouth and throat deposition and higher deep lunch deposition, leading to lower side effects, and higher efficacy and product acceptance by the user. Holding breath following the inhalation can also assist retention of much of the inhaled dose of nicotine in the respiratory tract.

Since smokers usually develop a strong "brand loyalty" to achieve a reproducible sensory perception, the repeatability of the desired aerosol plume geometry for a therapeutic form of nicotine is important also for a product to be accepted and hence effective for smoking cessation. A good plume quality is defined by a slow moving, homogenous, uniformly developing and consistent aerosol cloud.

In exemplary embodiments, the nicotine, or salt thereof, is delivered in the form of small inhalable droplets exhibiting a defined size distribution generated by the spray nozzle of the device. The desirable plume consists of fine droplets moving at a relatively low speed. In addition, a good plume is characterized by a consistent and uniformly developing shape.

In certain exemplary embodiments, the device administers droplets/particles in which at least 30%, 40%, or 50% of the emitted dose have an aerodynamic diameter finer than 5 microns. Such formulations are able to enter the lungs. In exemplary embodiments, at least 30%, 40%, or 50% of the emitted dose has an aerodynamic diameter that is finer than 3.99 microns, so as to assure that the majority of the emitted dose does in fact enters the lungs. Droplets/particles larger than 5 microns in contrast, are more likely to be deposited in the oropharynx and therefore are entrained into the system via the gastrointestinal (GI) tract. Drug absorption via the GI tract is generally slower than drug absorption via the lungs. Since it is apparently important that nicotine is absorbed fast to cause smoking cessation in cigarette smokers assist them to quit, the dose to the gastrointestinal tract should be minimized whereas the lung deposition should be maximized.

In other exemplary embodiments, suitable, for example, for tobacco smokers accustomed to getting their desired sensory perception by shallow puffing from, for example, cigars and pipes or certain types of vaping devices, similar breathing maneuvers can be employed to administer delivered with relatively larger particles or droplet to enhance buccal deposition and absorption. Accordingly, in certain embodiments, at least 30%, 40%, or 50% the emitted dose is contained in droplets with aerodynamic diameters > 4.0, or >5.0, or >10 microns.

### Soft-Mist Inhalers

Inhalers known in the art as soft-mist inhalers can be engineered as described herein and employed as a component of the presently disclosed device for delivery of API (e.g., nicotine).

According to the invention, the soft-mist inhaler is a soft mist inhaler employing intersecting jets to form aerosol droplets.

A non-limiting example of such an inhaler is a Respimat inhaler commercially available from Boehringer Ingelheim (Ingelheim am Rhein, Germany), and described in Development of Respimat Soft Mist Inhaler and its clinical utility in respiratory disorders, Med. Devices (Auckl.). 2011; 4:145-155, and Improving usability and maintaining performance: human-factor and aerosol-performance studies evaluating the new reusable Respimat inhaler, International Journal of COPD, Vol. 14:509-523 (March 2019).

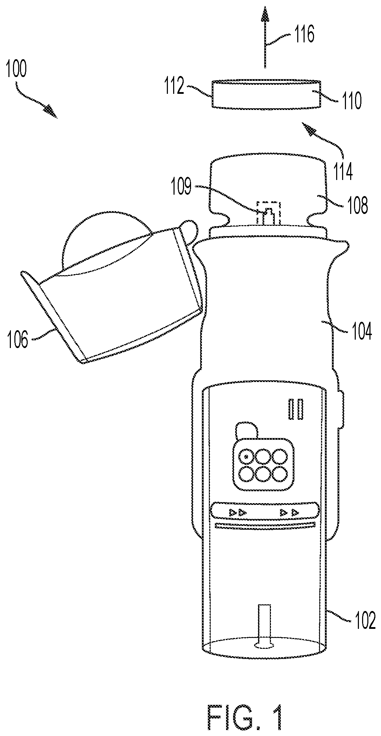

An exemplary device (100) for dispensing a cartridge (not shown) containing an API (e.g., nicotine) according to an embodiment of the invention as claimed is shown in FIG. 1. The device 100 includes a removable clear base 102, which is removed by the user prior to inserting the cartridge, and an inhaler body 104. This distal end of the inhaler body receives the cartridge, which is adapted to be received by the inhaler and secured upon clicked engagement. The clear base 102 is placed back into place, and the API is dispensed upon turning the clear base counterclockwise to load the API, opening the cap 106 and pressing an actuator button (located on the other side of the inhaler body as shown in FIG. 1) while inhaling from the mouthpiece 108. An insert 110 is placed inside the mouthpiece 108. A nozzle 109 is located at a distal location within the mouthpiece to deliver the aerosol.

The device shown in FIG. 1 is exemplary and not limiting. According to other exemplary embodiments, other soft mist inhalers can be employed. For example, AERx Essence^{®} (Aradigm) Aqueous Droplet Inhaler [ADI^{®}] (Pharmaero) Pre-filled syringe inhaler (Resyca BV), Pulmospray and Ecomyst (Aero Pump and Medspray) can be administered with formulations of the subject disclosure.

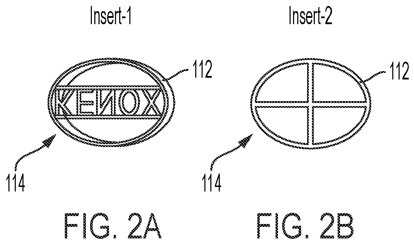

Each of the inserts are provided with an outer frame 112 with a cross-sectional shape adapted to be received snugly by the mouthpiece 108, oval in this exemplary embodiment. The distal end 114 of the insert 110 is further provided with obstructions in the airpath 116 of the device when placed into operation, shown best in FIGS. 2A-2H. FIGS. 2A-2H disclose inserts that can be incorporated as insert 110 according to exemplary embodiments, identified here as inserts 1-8 respectively. The designs, particularly, for example, Insert 1, can be used for shrinking compensation, in addition to delivering a larger portion of the emitted to the lungs, as opposed to the oropharynx.

More particularly, FIG. 2A discloses one version of insert 110 (Insert 1), in which the obstruction is defined by two cross bars offset from the major diameter of the oval, with the text "KENOX" centered about the airpath of the device and bound by the two cross bars. FIG. 2B discloses an alternative embodiment (Insert 2), in which the obstruction is defined by a simple cross, two bars respectively disposed about the major and diameters of the oval, each bar having a width in this exemplary embodiment of about 2 mm.

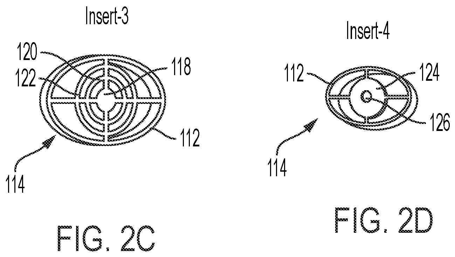

FIG. 2C discloses an alternative embodiment (Insert 3), in which the obstruction is defined by a central obstructing disk 118 having a diameter of about 3 mm, with a first aperture ring 120 being spaced about 1 mm from the obstructing disk, and a second aperture ring 122 being spaced about 1 mm from the first aperture ring. Two cross bars are also provided as with Insert 2, but provided up to the central obstructing disk 118. FIG. 2D discloses an alternative embodiment (Insert 4), in which the obstruction is defined by a central, relatively larger obstructing disk 124 having a diameter of about 10 mm, but with a central orifice 126 provided centrally about the obstructing disk. The central orifice 126 has a diameter of about 3 mm. Two cross bars are also provided as with Insert 2, but provided up the central obstructing disk 124.

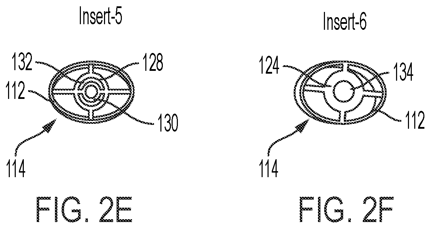

FIG. 2E discloses an alternative embodiment (insert 5). Insert 5 is similar to Insert 4, in which the obstruction is similarly defined by a central, relatively larger obstructing disk 128 having a diameter of about 10 mm, with a central orifice 130 provided centrally about the obstructing disk. The central orifice 130 has a diameter of about 3 mm. The obstruction is distinguished from Insert 4 in that Insert 5 is further defined by a second aperture 132 cut into the obstructing disk 128, which is a 1 mm width ring spaced 1 mm radially from the central orifice 130. Two cross bars are also provided as with Insert 2, but with the cross bar along the major axis provided up to the central orifice 130 and the cross bar along the minor axis provided up to the obstructing disk 128.

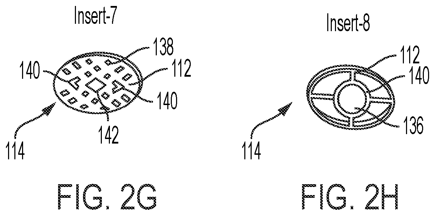

FIG. 2F discloses an alternative embodiment (insert 6). Insert 6 is similar to Insert 4 with a obstructing disk 124 having a diameter of about 9mm. Insert 6 is provided with a central orifice 134 having a diameter of 5 mm, instead of 3 mm in Insert 4. FIG. 2H discloses an alternative embodiment (Insert 8). Insert 8 is similar to Insert 4, except that the central orifice 136 is 9mm. Two cross bars are also provided in Inserts 6 and 8, as with Insert 2, but provided up to the central obstructing disk.

FIG. 2G discloses an alternative embodiment (Insert 7), in which the obstruction is defined by a plurality of shaped orifices 138 located equally spaced along the outer perimetry of the insert. A pair of relatively larger, arrowhead shaped orifices 140 are provided along the major axis spaced from a central diamond shaped orifice 143. The orifices are sized such as to provide obstructions along 50% of the total mouthpiece cross-sectional area along airpath 116 (FIG. 1).

In other exemplary embodiments, other mechanisms are employed to create a slow-moving gentle mist aerosol from a formulation of the subject disclosure (e.g., a solution). These mechanisms include, but are not limited to, impinging jets, Rayleigh instability, piezoelectric vibrations, thermoelectrics and electrohydrodynamics.

### EXAMPLES

The present disclosure will be further illustrated by the following non-limiting Examples. These Examples are understood to be exemplary only, and they are not to be construed as limiting the scope of the one or more embodiments, and as defined by the appended claims.

### Example 1

A number of different nicotine solutions for oral inhalation were tested to optimize the delivery of nicotine (Table 1). The target compositions were prepared with the view to deliver approximately 0.1, 0.25 and 0.5 mg of nicotine base per puffin ~ 11 µL of liquid to be consistent with the metering and delivered volumes per dose from the Respimat^{™} device.

The purpose of this development was to find the optimum combination of formulation parameters and device design to deliver an adequate dose of nicotine in a single breath to the small airways and alveoli for rapid absorption to reduce craving for cigarettes while minimizing deposition in the oropharynx and large airways to reduce potential for irritation. The composition of the formulation, method of aerosolization and device design and components were carefully selected to minimize potential for adverse health effects.

**Table 1. Developmental Formulations overview**

| **Formulation** | **Nicotine Strength** | **Description** | **NFB* concentration (Theoretical)** |
|---|---|---|---|
| A | low | 0% EtOH | 9 mg/ml |
| B | low | 25% EtOH, | 9 mg/ml |
| C | low | 50% EtOH, | 9 mg/ml |
| D | low | 75% EtOH | 9 mg/ml |
| E | low | 0% EtOH | 9 mg/ml |
| F | low | 25% EtOH | 9 mg/ml |
| G | low | 75% EtOH | 9 mg/ml |
| H | mid | 75% EtOH 1N HCl | 22.5 mg/ml |
| I | high | 70% EtOH 1N HCl pH=2.5 | 45 mg/ml |
| J | high | 75% EtOH 2N HCl pH=1.5 | 45 mg/ml |
| K | mid | 75% EtOH 1N HCl pH=1.5 | 22.5 mg/ml |
| L | high | 70% EtOH 1N HCl pH=2.5 | 45 mg/ml |
| M | high | 75% EtOH 2N HCl pH=1.5 | 45 mg/ml |

| | | | |
|---|---|---|---|
| *NFB = Nicotine free base basis | | | |

After compounding, the assay of formulations was determined using internal RP-HPLC method. The assay results are presented in Table 2.

**Table 2. Representative assay results**

| Formulation | **Strength** | **%EtOH** | ***NFB Assay (mg/ml)** |
|---|---|---|---|
| A | low | 0% | 9.1 |
| B | low | 25% | 9.4 |
| C | low | 50% | 9.5 |
| D | low | 75% | 9.4 |
| K | mid | 75% | 22.9 |
| L | high | 70% (1N HCl) | 45.0 |
| M | high | 75% (2N HCl) | 44.1 |

| | | | |
|---|---|---|---|
| *NFB = Nicotine free base basis | | | |

During the development activities, a modified Respimat^{®} device was used. The modification was performed with respect to the mouthpiece insert which was retrofitted into Respimat^{®} devices.

To further reduce the oropharyngeal deposition, mouthpiece inserts were prototyped to selectively minimize the proportion of larger droplets exiting the mouthpiece of the device. The mouthpiece inserts were manufactured by a fused filament fabrication (FFF) 3D printing method. It is of note that FFF is sometimes also denoted fused deposition modelling (FDM). The inserts are designed to result in a snug fit, allowing for different positions in terms of distance from the spray nozzle.

Eight different inserts - i.e., Inserts 1-8 -- were tested. The insert designs are as depicted in FIGS. 2A-2H.

The inserts were then fitted into the device mouthpiece and subjected to visual assessment and droplet size distribution (DSD) based on laser diffraction. Based on the results of visual assessment and DSD determination, inserts 4, 5 and 7 were selected for further NGI testing.

The drug product comprising the device and cannister filled with a formulation, was characterized first in terms of visual quality assessment of the plume quality followed by assay of the formulation, DSD and APSD using various versions of devices based on the Respimat^{®}. The DSD was also assessed for various configurations resulting from placing the mouthpiece inserts at different distances from the spray nozzle.

### Analytical procedures

In this section, the analytical procedures used for the drug product characterization during the development are presented.

**Table 3. Overview of methods used for drug product characterization**

| **Test** | **Methodology** |
|---|---|
| Assay | HPLC |
| pH | Potentiometric |
| Droplet Size Distribution | Laser diffraction |
| Aerodynamic Particle size distribution | Cascade impaction (NGI) - USP <601> |
| Delivered dose | DUSA - USP <601> |

**Table 4. Droplet Size Distribution method details**

| **Parameter** | **Setting / Description** |
|---|---|
| Instrument | Malvern SprayTec equipped with the inhaler module without universal induction port |
| Hardware configuration | Inhalation cell |
| Flow | 15 liters per minute |
| Measurement type | Rapid |
| Data Acquisition rate | Different (0.5 - 2.5 - kHz) |
| Trigger condition | Different (transmission based <70% - 95%) |
| Events | 3 |
| Refractive index particle | 1.33 + i*0 |
| Refractive index dispersant | 1.00 + i*0 |

**Table 5. APSD method details**

| **Parameter** | **Setting / Description** |
|---|---|
| Instrument | NGI, Copley |
| Configuration | USP <601> Apparatus 5, cooled at 5°C for NLT 90 minutes |
| Pre-separator | No |
| Coating | No |
| Flow rate | 30 liters per minute |
| Actuations | 10 |
| Work up volumes (Diluent of NGI Assay method) | Insert: 5 ml |
| | Mouthpiece adapter : 10 ml |
| | Induction port : 20 ml |
| | Stages: 5 ml |
| | Filter:10 ml |
| Work up time | 10 min |

| | |
|---|---|
| NLT: Not Less Than | |

### Example 2

Initially, the plume was evaluated visually by firing the device after priming in an upright position. This evaluation was conducted using the low strength formulation. The results of the visual/qualitative assessment are presented in Table 6.

**Table 6. Visual/qualitative observations (low strength formulation)**

| **Insert number** | **0%EtOH** | **25 % EtOH** | **50%EtOH** | **75%EtOH** |
|---|---|---|---|---|
| 1 | x | x | x | x |
| 2 | x | x | x | x |
| 3 | x | x | x | x |
| 4 | ✔ | ✔ | ✔ | ✔ |
| 5 | ✔ | ✔ | ✔ | ✔ |
| 6 | ✔ | ✔ | ✔ | ✔ |
| 7 | ✔ | ✔ | ✔ | ✔ |
| 8 | ✔ | ✔ | ✔ | ✔ |

| | | | | |
|---|---|---|---|---|
| x: Plume quality - poor/distorted ✔: Plume quality - good | | | | |

The DSD of the formulations was determined using the laser diffraction The DSD was evaluated in triplicate (n=3) with regards to d10, d50, d90 and the span. Initially, the DSD was determined with the inserts being flush (top position) with the mouthpiece which corresponds to a distance between spray nozzle orifice and insert of 6 mm. The results including %RSDs are presented in Table 7,
Table 8, Table 9 and Table 10.

**Table 7. DSD results 0%EtOH (low strength formulation)**

| **Insert number** | **Insert position** | **d10 (µm/% RSD)** | **d50 (µm/%RSD)** | **d90 (µm/%RSD)** | **Span /%RSD** |
|---|---|---|---|---|---|
| None | NA | 1.77 / 4.4% | 4.91/8.1% | 9.87 / 9.1% | 1.65 / 3.4% |
| 4 | 6 mm | 1.85 / 4.4% | 4.39 / 1.6% | 8.61 / 2.7% | 1.54 / 3.1% |
| 5 | 6 mm | 1.81/1.0% | 4.20 / 2.1% | 8.31 / 3.1% | 1.55 / 2.2% |
| 6 | 6 mm | 1.92 /0.5% | 4.51 / 1.7% | 8.93 / 2.3% | 1.56 / 1.3% |
| 7 | 6 mm | 1.81 / 1.3% | *4.05* / *5.5%* | 8.00 / 7.2% | 1.53 / 4.0% |
| 8 | 6 mm | 1.99 / 2.7% | 4.37 / 1.9% | 8.50 / 2.3% | 1.49 / 2.3% |

**Table 8. DSD results 25%EtOH (low strength formulation, insert position 6 mm)**

| **Insert number** | **d10 (µm/%RSD)** | **d50 (µm/%RSD)** | **d90 (µm/%RSD)** | **Span /%RSD** |
|---|---|---|---|---|
| None | 1.82/ 1.3% | 4.07/2.2% | 7.97 / 2.2% | 1.51 / 0.4% |
| 4 | 1.53 / 2.9% | 3.94 / 8.6% | 8.24 / 12.1% | 1.70 / 7.3% |
| 5 | 1.49 / 13.1% | 3.99 / 4.5% | 8.68 / 12.5% | 1.80 / 13.8% |
| 6 | 1.72 / 2.1 % | 3.97 / 1.9% | 8.00 / 2.1% | 1.58 / 1.5% |
| 7 | 1.48 / 1.9% | 4.05 / 4.5% | 8.68/4.4% | 1.78 / 1.7% |
| 8 | 1.78 / 4.5% | 4.11 / 6.9% | 8.20 / 10.5% | 1.56 / 7.7% |

**Table 9. DSD results 50%EtOH (low strength formulation insert position 6 mm)**

| **Insert number** | **d10 (µm/%RSD)** | **d50 (µm/%RSD)** | **d90 (µm/%RSD)** | **Span /%RSD** |
|---|---|---|---|---|
| None | 1.68 / 1.5% | 3.61/1.6% | 7.12 / 1.4% | 1.51 / 0.2% |
| 4 | 1.62 / 2.5% | 3.78 / 1.0% | 7.74 / 0.3% | 1.62 / 2.0% |
| 5* | 1.56 / | 3.69 / - | 7.57 / - | 1.63 / - |
| 6 | 1.67 / 0.7% | 3.74 / 3.3% | 7.50 / 4.7% | 1.56 / 2.5 |
| 7 | 1.45 / 0.1% | 3.52 / 0.1% | 7.48 / 0.1% | 1.72 / 0.1% |
| 8 | 1.65 / 2.2% | 3.68 / 5.7% | 7.41 / 7.1% | 1.56 / 2.9% |

| | | | | |
|---|---|---|---|---|
| *: note that only two determinations are available and therefore no %RSD was calculated | | | | |

**Table 10. DSD results 75%EtOH (low strength formulation insert position 6 mm)**

| **Insert number** | **d10 (µm/%RSD)** | **d50 (µm/%RSD)** | **d90 (µm/%RSD)** | **Span /%RSD** |
|---|---|---|---|---|
| None | 1.19/5.3% | 2.58 / 4.6% | 5.42/3.5% | 1.64 / 1.6% |
| 4 | 0.99 / 2.6% | 2.30/ 1.4% | 5.06 / 0.7% | 1.77/ 1.2% |
| 5 | 1.01 / 9.5% | 2.27 / 9.3% | 4.93/6.8% | 1.73 / 3.1% |
| 6 | not tested | not tested | not tested | not tested |
| 7 | 0.97/4.2% | 2.25 / 4.8% | 4.97 / 4.2% | 1.78 / 0.6% |
| 8 | not tested | not tested | not tested | not tested |

### Example 3

The DSD was replaced by aerodynamic particle size distribution (APSD) determined using cascade impactor (CI).

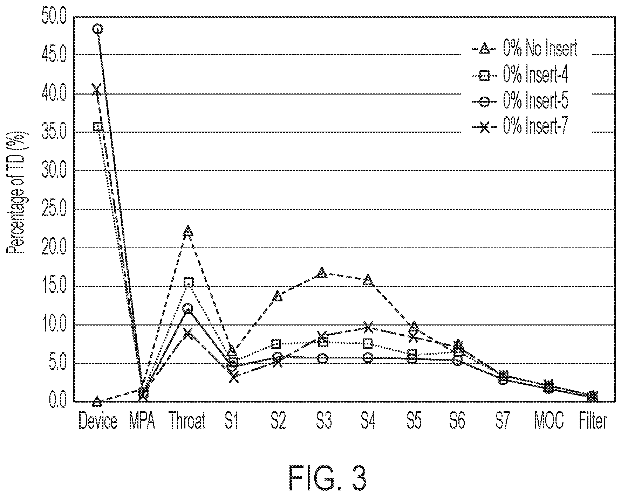

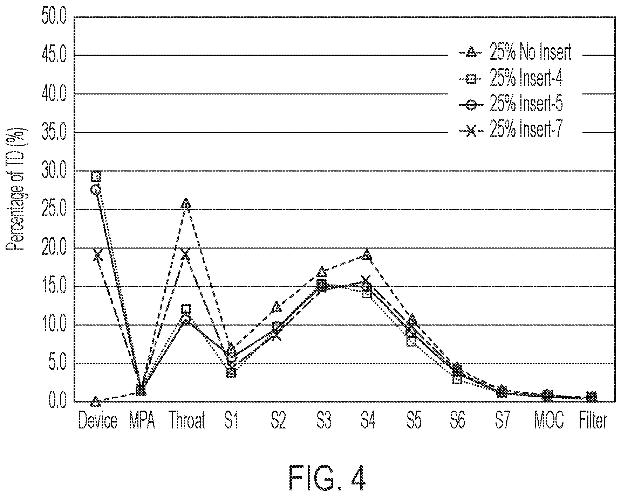

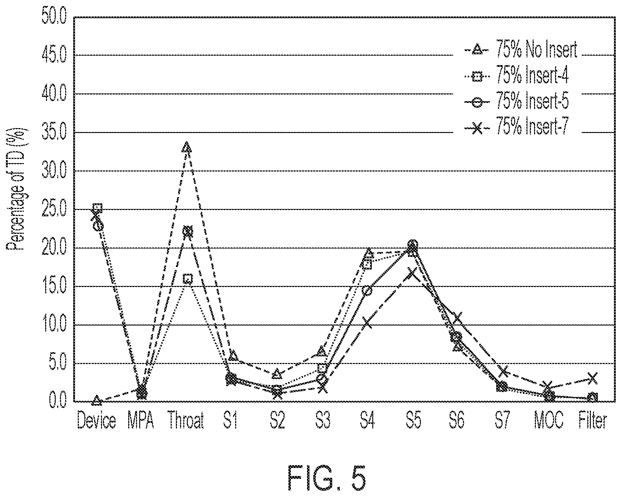

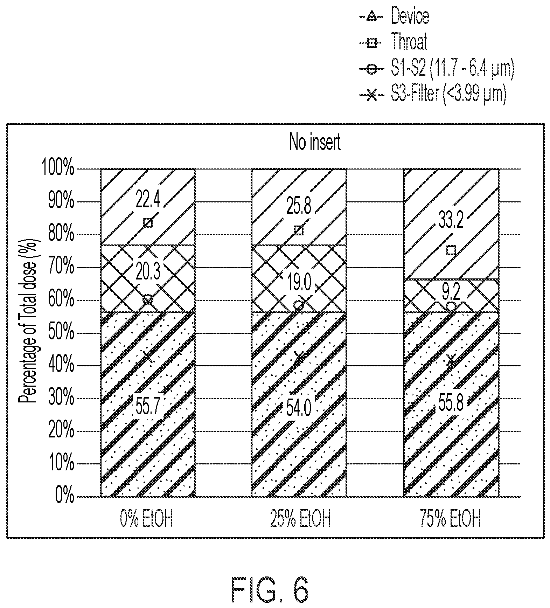

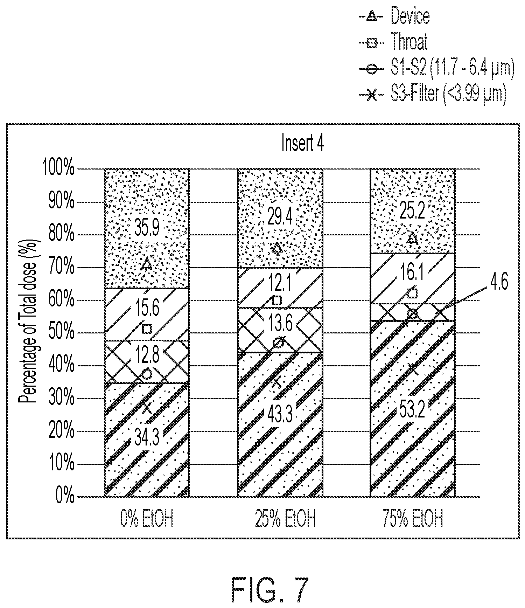

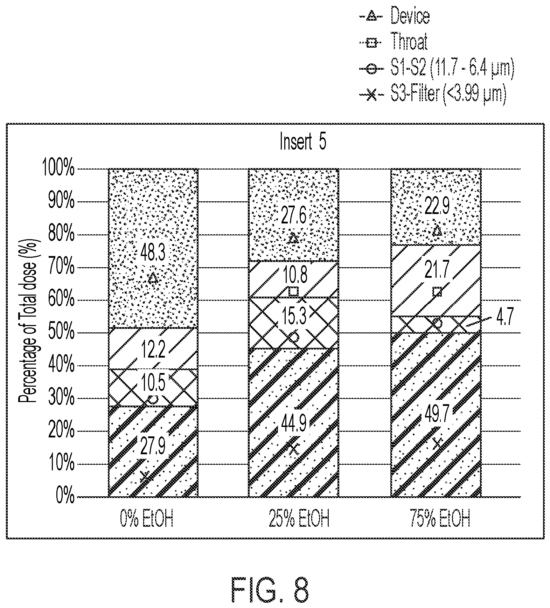

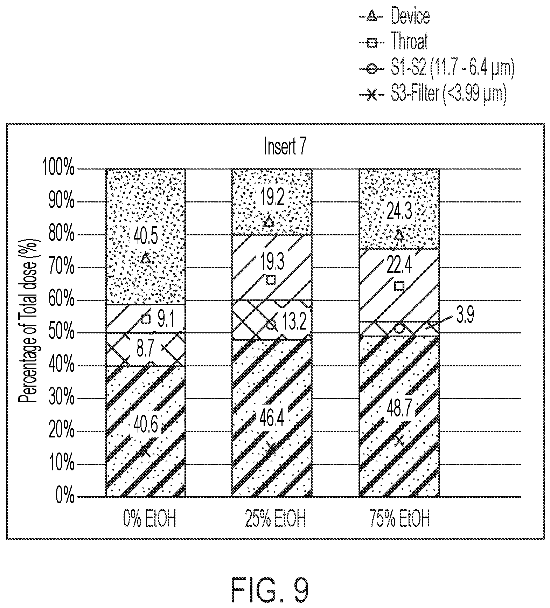

Based on the DSD results, inserts 4, 5 and 7 were selected for APSD testing using 0%, 50% and 75% EtOH (v/v) with the insert being flush (top position) in the mouthpiece initially. The fine particle dose FPD, fine particle fraction FPF, GSD and MMAD were calculated using Copley's CITDAS version 3.10. For a better comparison, the graphical results were normalized against the total dose recovered, see FIG. 3, FIG. 4, and FIG. 5. And lastly, the results were calculated as percentage of different size classes and their contribution to the total dose emitted ex device, see FIG. 6, FIG. 7, FIG. 8 and FIG. 9.

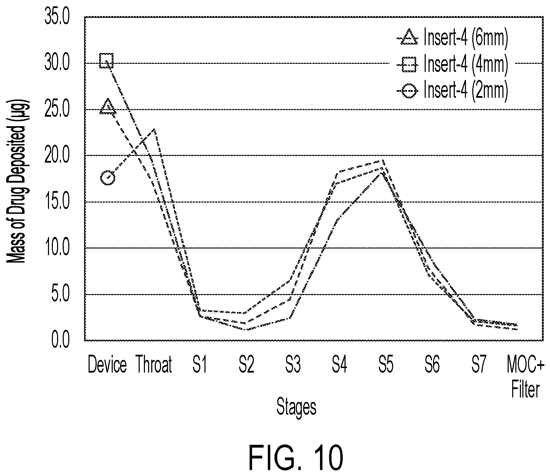

The influence of the insert position was then assessed using 75%EtOH formulation. Inserts 4, 5 and 7 were placed at a distance of 6 or 4 or 2 mm from the spray nozzle orifice. The results are depicted in FIG. 10, FIG. 11, AND FIG. 12.

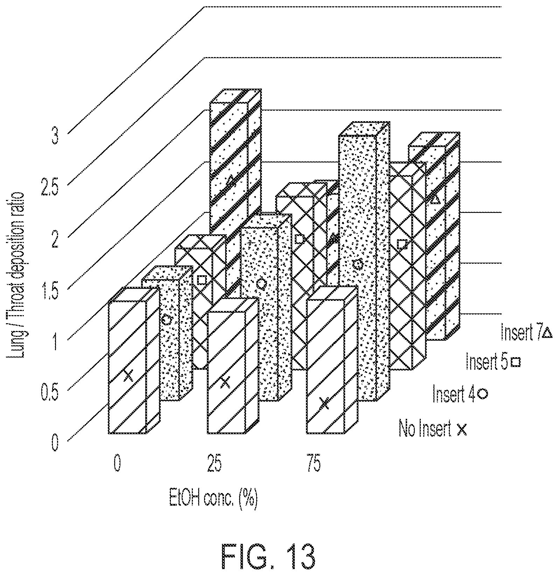

The results show that the mouthpiece inserts have a significant effect on the APSD. For example, if it is the delivered dose to the lungs per spray rather than the total dose emitted from the device that is preferred (e.g., to aid smoking cessation in cigarette smokers), then approximating the "lung dose" with the percentage of the inhalable fraction (here defined as the drug deposited from stage 3 to the filter of the NGI cascade impactor) compared to the total dose ex device (without device deposition) is an important performance indicator. When combined with a development target of minimal throat deposition, using 75%EtOH and insert 4 is the optimal combination (FIG. 13). For the sake of clarity, in the context of the experiments reported here, the terms "lung deposition" and "lung dose" are used to describe the mass deposited at 4.0 microns and lower cut-off stages of the NGI whereas the "Throat" deposition denotes the mass above 4.0 microns cut-off stages.

Thus, the combination of Insert 4 in the flush position (6 mm) and 75% EtOH (low strength nicotine) results in a "throat" deposition of approximately 21% of the total dose whilst exhibiting approximately 53% of the total dose (ex-device) deposited in the "lung dose" size range of <4.0 microns.

It is further of note that, compared to a purely aqueous solution, using 75% EtOH is thought to act as bacteriostatic vehicle thereby mitigating the risk of microbial growth in the product.

### Example 4

The Influence of inserts at different positions using low strength formulation was investigated.

Using APSD testing by cascade impaction. This showed a significant influence of the distance between spray nozzle and the insert. It can be observed that different inserts react differently to the distance between spray nozzle and insert.

In particular, for insert 4 (FIG. 10), the "throat deposition" increases when the insert is brought to a distance of 4 mm from the nozzle. Also, a reduction in stage 4 deposition can be observed. When the insert 4 is brought even closer to the nozzle (2 mm), the "throat deposition" increases further and an APSD similar to 6 mm (i.e. flush/top position) can be observed.

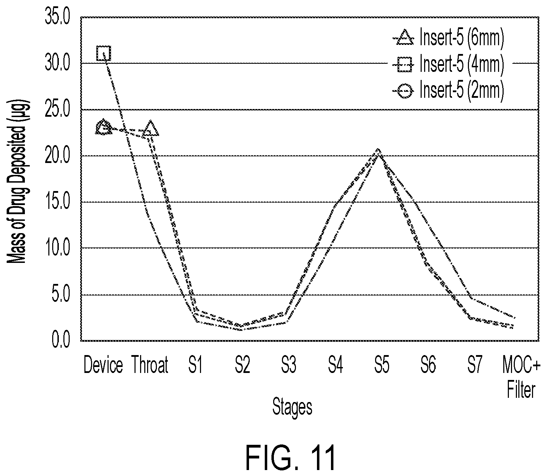

For insert 5 brought to 4 mm distance (FIG. 11), the throat deposition is further decreased compared to 6 mm while the deposition in stage 6 and onwards is increased. When the insert is brought to 2 mm distance, a similar APSD compared to 6 mm can be observed.

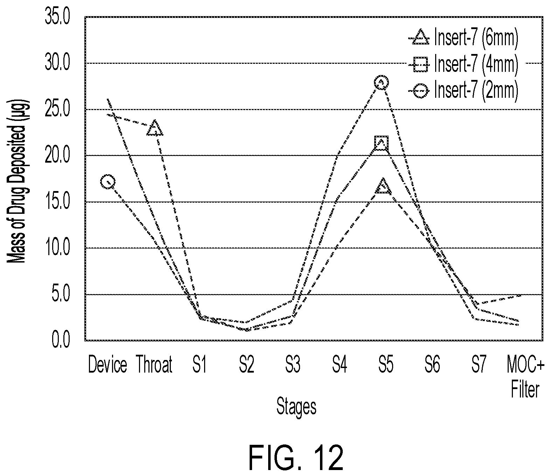

For insert 7 (FIG. 12), the throat deposition is further decreased whilst the deposition in stage 4 to stage 6 is increased at an insert position of 4 mm. However, contrary to insert 4 and insert 5, the throat deposition further decreases while the APSD in stage 4 and onwards increases even further with the insert distance of 2 mm.

Overall, insert 7 at 2 mm distance from the nozzle is the preferred combination when the design goal is to cause the therapeutic effect by nicotine droplet or particle deposition predominantly in the small airways and alveoli while minimizing the deposition in the oropharynx.

### Example 5

The delivered dose (DD) of nicotine compositions were determined in an abbreviated form following USP<601> using formulations containing 0%, 25%, 50% and 75% (v/v) EtOH for the low strength as well as for the lead formulations of mid and high strength. Briefly, the DD was determined by means of Dose Unit Sampling Apparatus (DUSA) tubes using a flowrate of 30 lpm and 10 shots in one DUSA in duplicate, i.e. n= 2 x 10. The delivered dose is presented as drug substance delivered ex-device, i.e. without device/insert deposition. In addition, the devices were weighed before and after the 10 actuations to determine the shot weight. The results are presented in Table 11.

**Table 11. Delivered dose and shot weights of formulations (insert 7 at 2 mm)**

| **Formulation** | **Nicotine Strength** | **%EtOH (v/v)** | **Delivered dose of NFB ex device(n=2x10) (µg (min-max))** | **Average Shot weight (mg)** |
|---|---|---|---|---|
| N | low | 0 | 127.6 (126.8 - 128.4) | 15.2 |
| O | low | 25 | 120.6 (119.9 - 121.2) | 14.2 |
| P | low | 50 | 116.2 (116.0 - 116.4) | 13.3 |
| Q | low | 75 | 119.6 (119.4 - 119.7) | 14.3 |
| G | low | 75 | 86.7 (82.7 - 90.6) | 14.2 |
| H | mid | 75 (1N HCl) | 207.0 (197.4 - 215.6) | 14.1 |
| I* | High single analysis of 10 shots | 70 (1N HCl) | 402.2 | 13.5 |
| J | high | 75 (2N HCl) | 406.0 (399.9 - 412.0) | 14.0 |

| | | | | |
|---|---|---|---|---|
| NFB: nicotine free base *: The delivered dose for batch I was determined by single analysis of 10 shots | | | | |

### Example 6

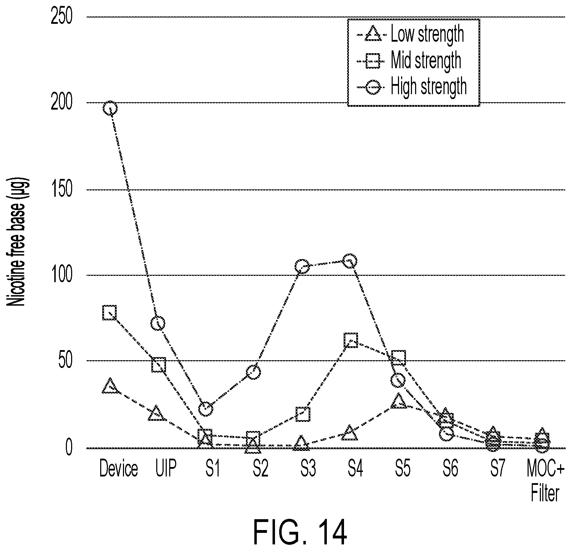

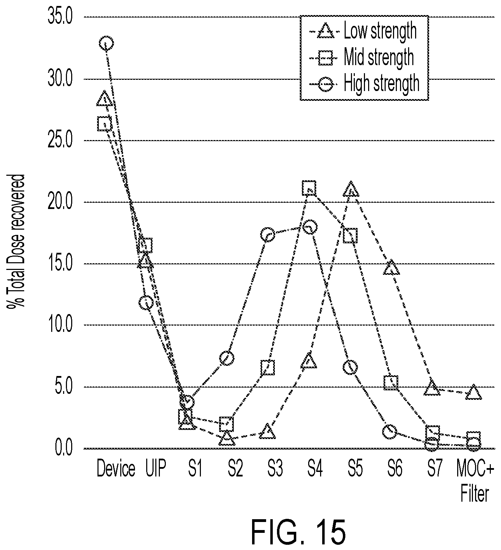

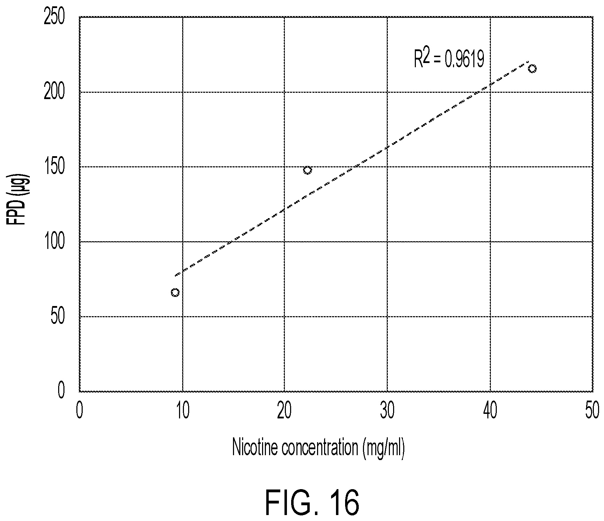

The formulations of the low (Formulation G), mid (Formulation H) and high (Formulation J) strengths were compared with insert 7 placed 2 mm from the nozzle. The FPD, FPF, GSD and MMAD were calculated using Copley's CITDAS version 3.10. A summary of key parameters is presented in Table 12. An overlay of the APSD of the different strengths in both, absolute and normalized terms, is depicted in FIG. 14 and FIG. 15 respectively. The delivered doses and the FPD ex-device are depicted in Table 12 and FIG. 16 respectively, as functions of the concentration of the formulation. Table 12. Summary of key parameters of low, mid and high strength formulations insert 7, 2 mm from nozzle

| **Parameter** | **Low strength Formulation G** | **Mid Strength Formulation H** | **High Strength Formulation J** |
|---|---|---|---|
| Assay* (mg/ml) | 9.2 | 22.3 | 44.0 |
| MMAD (µm (min - max)) | 1.5 (1.49 - 1.49) | 2.5 (2.52 - 2.52) | 3.8 (3.81 - 3.84) |
| GSD (min - max) | 1.8 (1.77 - 1.77) | 1.8 (1.79 - 1.80) | 1.7 (1.69 - 1.72) |
| FPD (µg (min - max)) | 66.4 (66.08 - 66.68) | 143.7 (143.66 - 151.23) | 223.4 (215.6 - 231.3) |
| FPF (% (min - max)) | 73.9 (73.52 - 74.25) | 67.2 (67.19 - 67.51) | 57.4 (57.25 - 57.48) |
| Delivered dose (ex-device w/o insert deposition) USP<601> (µg /(min-max)) | 86.7 (82.7 - 90.6) | 207.0 (197.4 - 215.6) | 406.0 (399.9 - 412.0) |

| | | | |
|---|---|---|---|
| *: The assay was determined by single measurement | | | |

In Table 13, a comparison of results of the aerosol characterizations using an aqueous formulation / no insert; 75% ethanol-water (v/v) for the three different strengths using insert 7 position 2 mm from the nozzle, is presented.

**Table 13. APSD and delivered dose summary comparing three different strengths to an aqueous control formulation with no insert.**

| **Parameter** | **Low strength Aqueous/no insert (Formulation A - control)** | **Low strength Formulation G** | **Mid Strength Formulation H** | **High Strength Formulation J** |
|---|---|---|---|---|
| MMAD (µm) | 3.8 | 1.5 | 2.5 | 3.8 |
| GSD | 2.2 | 1.8 | 1.8 | 1.7 |
| FPD (µg) | 58.3 | 66.4 | 143.7 | 223.5 |
| FPF (%) | 47.4 | 73.9 | 67.2 | 57.4 |
| Delivered dose (ex- | 127.6** | 86.7 | 207.0 | 406.0 |
| device) USP<601> (µg)* | | | | |

| | | | | |
|---|---|---|---|---|
| * Delivered dose was determined in duplicate using 10 shots in one DUSA tube each, data presented is without mouthpiece insert deposition, i.e., ex-device. ** The delivered dose data was determined using batch Formulation N. | | | | |

These results demonstrate that the currently disclosed subject matter provides different ratios of lung doses vs. doses delivered to the upper and central airways to be delivered using different combinations of ethanol and water, different types of mouthpiece inserts and their distance from the aerosol generating nozzle. In particular, the examples illustrate the exemplary formulations, insert design and its distance in the mouthpiece from the aerosolizing nozzle to deliver nicotine for therapeutic purposes such as for assistance to cigarette smokers to quit, prevent relapse and relieve craving for cigarettes.

## Claims

1. An inhaler (100) for delivering an active pharmaceutical ingredient, API, in aerosol form, wherein the inhaler is a soft mist inhaler employing intersecting jets to form aerosol droplets, the inhaler comprising:
(a) a container that includes an API and one or more pharmaceutically acceptable carriers, wherein the API is in an aqueous solution;
(b) a nozzle (109), valve or outlet in communication with the container to deliver an aerosol;
(c) an insert (110) or number of inserts located downstream of the nozzle, valve or outlet, wherein the insert provides obstructions in the airpath of the inhaler downstream of the nozzle, valve or outlet when placed into operation.

2. The inhaler (100) of claim 1, wherein the insert (110) is located from about 1 to about 10 mm downstream from the nozzle (109), valve, or outlet.

3. The inhaler (100) of claim 1, wherein the insert (110) includes a centrally located orifice (126, 130, 134, 136, 143).

4. The inhaler (100) claim 1, wherein the insert (110) provides obstructions along from about 10% to about 90% of a total cross-sectional area along an airpath downstream of the nozzle (109), valve, or outlet.

5. The inhaler (100) of claim 1, wherein the aqueous solution includes an alcohol.

6. The inhaler (100) of claim 5, wherein the alcohol is ethanol.

7. The inhaler (100) of claim 1, wherein the inhaler emits an aerosol dose including the API in which at least 30%, of the emitted dose has aerodynamic diameters between 0.5 - 3.5 micrometers.

8. The inhaler (100) of claim 1, wherein the aerosol dose is emitted at inspiratory flow rates between 5 - 140 L/min.

9. The inhaler (100) of claim 1, wherein at least 40% of the emitted dose is contained in droplets with aerodynamic sizes less than about 4 microns.

10. The inhaler (100) of any one of claims 1 to 9, wherein the API is nicotine or a salt thereof.

11. The inhaler (100) of claim 10, wherein the API is in the form of a nicotine salt.

12. The inhaler (100) of claim 11, wherein the nicotine salt is a citrate salt or a sulphate salt.

13. The inhaler (100) of claim 10, wherein from about 0.01 mg to about 1 mg of nicotine (free base basis) is delivered per actuation.

14. The inhaler (100) of one of claims 1-13, wherein the one or more inserts (110) are located in a mouthpiece (108) of the inhaler.

15. The inhaler (100) of claim 14, wherein each of the inserts (110) are provided with an outer frame (112) with a cross-sectional shape adapted to be received snugly by the mouthpiece (108), in particular oval, and/or wherein the obstructions are provided in the distal end (114) of the insert.

## Patentansprüche

1. Inhalator (100) zur Abgabe eines pharmazeutischen Wirkstoffs, API, in Aerosolform, wobei der Inhalator ein Feinnebel-Inhalator ist, der sich kreuzende Strahlen zur Bildung von Aerosoltröpfchen einsetzt, wobei der Inhalator Folgendes umfasst:
(a) einen Behälter, der einen API und einen oder mehrere pharmazeutisch annehmbare Träger enthält, wobei sich der API in einer wässrigen Lösung befindet;
(b) eine Düse (109), ein Ventil oder einen Auslass in Verbindung mit dem Behälter, um ein Aerosol abzugeben;
(c) einen Einsatz (110) oder eine Anzahl von Einsätzen, die sich stromabwärts der Düse, des Ventils oder des Auslasses befinden,
wobei der Einsatz im Luftweg des Inhalators stromabwärts der Düse, des Ventils oder des Auslasses Hindernisse bereitstellt, wenn er in Betrieb genommen wird.

2. Inhalator (100) nach Anspruch 1, wobei sich der Einsatz (110) etwa 1 bis etwa 10 mm stromabwärts von der Düse (109), dem Ventil oder dem Auslass befindet.

3. Inhalator (100) nach Anspruch 1, wobei der Einsatz (110) eine zentral gelegene Öffnung (126, 130, 134, 136, 143) beinhaltet.

4. Inhalator (100) nach Anspruch 1, wobei der Einsatz (110) entlang etwa 10 % bis etwa 90 % der gesamten Querschnittsfläche entlang eines Luftwegs stromabwärts der Düse (109), des Ventils oder des Auslasses Hindernisse bereitstellt.

5. Inhalator (100) nach Anspruch 1, wobei die wässrige Lösung einen Alkohol enthält.

6. Inhalator (100) nach Anspruch 5, wobei der Alkohol Ethanol ist.

7. Inhalator (100) nach Anspruch 1, wobei der Inhalator eine Aerosoldosis freisetzt, die den Wirkstoff enthält, wobei mindestens 30 % der freigesetzten Dosis aerodynamische Durchmesser zwischen 0,5-3,5 Mikrometern aufweisen.

8. Inhalator (100) nach Anspruch 1, wobei die Aerosoldosis bei Inspirationsflussraten zwischen 5-140 I/min freigesetzt wird.

9. Inhalator (100) nach Anspruch 1, wobei mindestens 40 % der freigesetzten Dosis in Tröpfchen mit aerodynamischen Größen von weniger als etwa 4 Mikrometern enthalten sind.

10. Inhalator (100) nach einem der Ansprüche 1 bis 9, wobei der API Nikotin oder ein Salz davon ist.

11. Inhalator (100) nach Anspruch 10, wobei der API in Form eines Nikotinsalzes vorliegt.

12. Inhalator (100) nach Anspruch 11, wobei das Nikotinsalz ein Citratsalz oder ein Sulfatsalz ist.

13. Inhalator (100) nach Anspruch 10, wobei pro Betätigung etwa 0,01 mg bis etwa 1 mg Nikotin (auf Basis der freien Base) abgegeben werden.

14. Inhalator (100) nach einem der Ansprüche 1-13, wobei sich der eine oder die mehreren Einsätze (110) in einem Mundstück (108) des Inhalators befinden.

15. Inhalator (100) nach Anspruch 14, wobei jeder der Einsätze (110) mit einem Außenrahmen (112) mit einer Querschnittsform versehen ist, die dazu angepasst ist, passgenau vom Mundstück (108) aufgenommen zu werden, insbesondere oval, und/oder wobei die Hindernisse im distalen Ende (114) des Einsatzes bereitgestellt sind.

## Revendications

1. Inhalateur (100) destiné à administrer une substance active pharmaceutique, API, sous forme d'aérosol, dans lequel l'inhalateur est un inhalateur à brume douce employant des jets intersectants pour former des gouttelettes d'aérosol, l'inhalateur comprenant :
(a) un récipient qui comporte une API et un ou plusieurs excipients pharmaceutiquement acceptables, dans lequel l'API est dans une solution aqueuse ;
(b) une buse (109), une valve ou une sortie en communication avec le récipient pour administrer un aérosol ;
(c) un insert (110) ou un nombre d'inserts situés en aval de la buse, de la valve ou de la sortie,
dans lequel l'insert fournit des obstructions dans le trajet d'air de l'inhalateur en aval de la buse, de la valve ou de la sortie lorsqu'il est mis en fonctionnement.

2. Inhalateur (100) selon la revendication 1, dans lequel l'insert (110) est situé d'environ 1 à environ 10 mm en aval de la buse (109), de la valve, ou de la sortie.

3. Inhalateur (100) selon la revendication 1, dans lequel l'insert (110) comporte un orifice situé au centre (126, 130, 134, 136, 143).

4. Inhalateur (100) selon la revendication 1, dans lequel l'insert (110) fournit des obstructions sur environ 10 % à environ 90 % d'une surface de section transversale totale le long d'un trajet d'air en aval de la buse (109), de la valve, ou de la sortie.

5. Inhalateur (100) selon la revendication 1, dans lequel la solution aqueuse comporte un alcool.

6. Inhalateur (100) selon la revendication 5, dans lequel l'alcool est l'éthanol.

7. Inhalateur (100) selon la revendication 1, dans lequel l'inhalateur émet une dose d'aérosol comportant l'API où au moins 30 %, de la dose émise a des diamètres aérodynamiques entre 0,5 et 3,5 micromètres.

8. Inhalateur (100) selon la revendication 1, dans lequel la dose d'aérosol est émise à des débits inspiratoires entre 5 et 140 L/min.

9. Inhalateur (100) selon la revendication 1, dans lequel au moins 40 % de la dose émise est contenue dans des gouttelettes avec des tailles aérodynamiques inférieures à environ 4 microns.

10. Inhalateur (100) selon l'une quelconque des revendications 1 à 9, dans lequel l'API est la nicotine ou un sel de celle-ci.

11. Inhalateur (100) selon la revendication 10, dans lequel l'API est sous la forme d'un sel de nicotine.

12. Inhalateur (100) selon la revendication 11, dans lequel le sel de nicotine est un sel de citrate ou un sel de sulfate.

13. Inhalateur (100) selon la revendication 10, dans lequel d'environ 0,01 mg à environ 1 mg de nicotine (exprimé en base libre) est administré par actionnement.

14. Inhalateur (100) selon l'une des revendications 1 à 13, dans lequel le ou les inserts (110) sont situés dans un embout buccal (108) de l'inhalateur.

15. Inhalateur (100) selon la revendication 14, dans lequel chacun des inserts (110) est pourvu d'un cadre externe (112) avec un profil en section transversale adapté pour être reçu de manière ajustée par l'embout buccal (108), en particulier ovale, et/ou dans lequel les obstructions sont fournies dans l'extrémité distale (114) de l'insert.
